(19) **Europäisches Patentamt**
**European Patent Office**
**Office européen des brevets**

(11) **EP 1 759 698 A1**

(12) **EUROPEAN PATENT APPLICATION**

(43) Date of publication:
**07.03.2007 Bulletin 2007/10**

(51) Int Cl.:
***A61K 31/44*** *(2006.01)* ***A61K 31/425*** *(2006.01)*

(21) Application number: **06124048.7**

(22) Date of filing: **12.11.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **12.11.1998 GB 9824893**

(62) Document number(s) of the earlier application(s) in accordance with Art. 76 EPC:
**99960293.1 / 1 135 128**

(71) Applicant: **SMITHKLINE BEECHAM CORPORATION**
**Philadelphia, PA 19103 (US)**

(72) Inventors:
- **Benincosa, Lisa**
  **Collegeville, PA 19426 (US)**
- **Jusko, William**
  **East Amherst, PA 14051 (US)**

(74) Representative: **Rutter, Keith**
**GlaxoSmithKline**
**Corporate Intellectual Property (CN9.25.1)**
**980 Great West Road**
**Brentford, Middlesex TW8 9GS (GB)**

Remarks:
This application was filed on 14 - 11 - 2006 as a divisional application to the application mentioned under INID code 62.

(54) **Novel method of treatment**

(57) A method for the treatment of type 2 diabetes mellitus and conditions associated with diabetes mellitus, which method comprises the administration to a human or non-human mammal in need thereof, of an effective non-toxic amount of an insulin sensitiser so as to provide a plasma concentration of the insulin sensitiser of at least a threshold level (the "Threshold Plasma Concentration") from within the range of effective plasma levels of the insulin sensitiser; compositions for use in such a method; and methodology for determining plasma concentrations of active agent used in such a method.

EP 1 759 698 A1

**Description**

This invention relates to a novel method of treatment, in particular to a method for the treatment of Type 2 diabetes mellitus and conditions associated with diabetes mellitus and a pharmaceutical composition for use in such a method.

European Patent Application, Publication Number 0,306,228 relates to certain thiazolidinedione derivatives disclosed as having antihyperglycaemic and hypolipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter 'Compound (I)'). WO94/05659 discloses certain salts of Compound (I) including the maleate salt at example 1 thereof.

Compound (I) is an example of a class of anti-hyperglycaemic agents known as 'insulin sensitisers'. In particular Compound (I) is a thiazolidinedione insulin sensitiser.

European Patent Applications, Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

Another series of compounds generally recognised as having insulin sensitiser activity are those typified by the compounds disclosed in International Patent Applications, Publication Numbers WO93/21166 and WO94/01420. These compounds are herein referred to as 'acyclic insulin sensitisers'. Other examples of acyclic insulin sensitisers are those disclosed in United States Patent Number 5232945 and International Patent Applications, Publication Numbers WO92/03425 and WO91/19702.

Examples of other insulin sensitisers are those disclosed in European Patent Application, Publication Number 0533933, Japanese Patent Application Publication Number 05271204 and United States Patent Number 5264451.

The above mentioned publications are incorporated herein by reference.

It is now surprisingly indicated that the particular plasma concentrations of an anti-diabetic agent, such as Compound (I), which provide effective glycaemic control, indeed an optimum effect on glycaemic control, can be determined. This therefore enables optimization of the dosing regimen for the anti-diabetic agent for a given dosing interval. Pharmaceutical compositions which provide plasma concentrations of an anti-diabetic agent, such as Compound (I), at these particular concentrations, especially over an extended period of time, are also envisaged by this invention.

Accordingly, in a first aspect, the present invention provides a method for the treatment of Type 2 diabetes mellitus and conditions associated with diabetes mellitus, which method comprises the administration to a human or non-human mammal in need thereof, of an effective non-toxic amount of an insulin sensitiser, such as Compound (I), so as to provide a plasma concentration of the insulin sensitiser of at least a threshold level from within the range of effective plasma levels of the insulin sensitiser (hereinafter referred to as the 'Threshold Plasma Concentration').

The Threshold Plasma Concentration is suitably within the range of from 40 to 200ng/nL including 50 to 200ng/nL, including 50 to 120ng/mL, 60 to 120ng/mL, 90 to 110ng/mL or 95 to 105ng/mL.

A suitable minimum Threshold Plasma Concentration (hereinafter 'Minimum Threshold Plasma Concentration') is the SC50 concentration of the particular insulin sensitiser, which for Compound (I) is within the range of 40 to 65 ng/mL, more suitably 41.1 to 61.7, for example 50 or, more suitably, 51.4ng/mL.

A preferred Threshold Plasma Concentration (hereinafter 'Preferred Threshold Plasma Concentration') is twice the SC50 concentration, which for Compound (I) is in the range of 80 to 130 ng/mL, more suitably 82.2 to 123.4, for example 100 ng/mL or 102.8 ng/mL.

The invention particularly envisages treatments wherein the plasma concentration of the insulin sensitiser remains substantially within the range of concentrations from the Minimum Threshold Plasma Concentration to the Preferred Threshold Plasma Concentration, that is for Compound (I) within the range of from 40 to 130 ng/mL, more suitably 41.1ng/mL to 123.4 ng/mL, for example 50ng/mL to 100ng/mL or 51.4ng/mL to 102.8 ng/mL.

The invention also particularly envisages treatments wherein the plasma concentration of the insulin sensitiser remains substantially within the range of concentrations from the Minimum Threshold Plasma Concentration to a level at or above the Preferred Threshold Plasma Concentration, that is for Compound (I) within the range of from 40ng/mL to a level at or above 130 ng/mL, more suitably 41.1ng/mL to a level at or above 123.4 ng/mL, for example 50ng/mL to 100ng/mL or 51.4 ng/mL to a level at or above 102.8 ng/mL.

In its preferred form, the invention provides a treatment wherein the plasma concentration of the insulin sensitiser remains substantially at or above the Preferred Threshold Plasma Concentration, that is for Compound (I), substantially at or above 100ng/mL, especially substantially at or above 102.8ng/mL.

A suitable thiazolidinedione insulin sensitiser is Compound (I).

Other suitable thiazolidinedione insulin sensitisers include 5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone), 5-[4-[(1-methylcyclohexyl)methoxy]benzyl] thiazolidine-2,4-dione (or ciglitazone), 5-[4-[2-(5-ethylpyridin-2-yl)ethoxy]benzyl] thiazolidine-2,4-dione (or pioglitazone) or 5-[(2-benzyl-2,3-dihydrobenzopyran)-5-ylmethyl)thiazolidine-2,4-dione (or englitazone).

A particular thiazolidinedione insulin sensitiser is 5-[4-[2-(5-ethylpyridin-2-yl)ethoxy]benzyl] thiazolidine-2,4-dione (or pioglitazone). A particular thiazolidinedione insulin sensitiser is 5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone).

When the insulin sensitiser is Compound (I), the unit dose suitably comprises 2 to 12 or preferably 4 to 8 mg of Compound (I) in a pharmaceutically acceptable form.

Suitable unit dosages of other insulin sensitisers are those indicated in publications mentioned herein and include from 100 to 800mg of troglitazone such as 200, 400, 600 or 800mg and for pioglitazone from 5 to 50mg, including 10 to 40mg, such as 20, 30 or 40 mg and also including 15, 30 and 45mg of pioglitazone

As indicated above, the treatment of the invention is suitably effected by the administration of a pharmaceutical composition of the insulin sensitiser adapted so as to provide a plasma concentration of the insulin sensitiser of at least a Threshold Plasma Concentration of the insulin sensitiser.

Accordingly, in a further aspect, the invention also provides a pharmaceutical composition comprising an insulin sensitiser and a pharmaceutically acceptable carrier therefor, which composition is adapted to provide a plasma concentration of the insulin sensitiser of at least a Threshold Plasma Concentration of the insulin sensitiser, suitably over a sustained period of time.

Suitable modified release compositions are delayed, pulsed or sustained release compositions.

Accordingly, in a further aspect, the invention also provides a modified release pharmaceutical composition comprising an insulin sensitiser and a pharmaceutically acceptable carrier therefor, which composition is adapted to provide a plasma concentration of the insulin sensitiser of at least a Threshold Plasma Concentration of the insulin sensitiser, suitably over a sustained period of time.

Suitably the carrier is adapted to provide the provide a plasma concentration of the insulin sensitiser of at least a Threshold Plasma Concentration.

Suitably the modified release is a sustained release, for example providing effective release of active agents of at least a Threshold Plasma Concentration over a time period of up to 24 hours.

Suitably the modified release is a pulsed release, for example providing two pulses of release of active agents of at least a Threshold Plasma Concentration per 24 hours.

The invention particularly envisages compositions adapted to provide a plasma concentration of the insulin sensitiser which remains substantially within the range of concentrations from the Minimum Threshold Plasma Concentration to the Preferred Threshold Plasma Concentration, that is for Compound (I) within the range of from 40 to 130 ng/mL, more suitably 41.1 to 123.4 ng/mL, for example 50 to 100ng/mL or 51.4 to 102.8 ng/mL.

The invention also envisages compositions adapted to provide a plasma concentration of the insulin sensitiser which remains substantially at or above the Preferred Threshold Plasma Concentration, that is for Compound (I), substantially at or above 100ng/mL, especially substantially at or above 102.8ng/mL.

Suitably the composition is a unit dose composition.

Suitably, the Threshold Plasma concentration of the insulin sensitiser is maintained or exceeded over several hours, for example 12, 16 or 24 hours, per dose of insulin sensitiser.

Suitably, the treatment is such that the Threshold Plasma concentration of the insulin sensitiser is maintained or exceeded over a sustained period of time.

It will be understood that the insulin sensitiser, such as Compound (I), is administered in a pharmaceutically acceptable form, including pharmaceutically acceptable derivatives such as pharmaceutically acceptable salts, esters and solvates thereof, as appropriate of the relevant pharmaceutically active agent. It will be understood that all pharmaceutically acceptable forms of the active agents per se are encompassed by this invention.

Suitable pharmaceutically acceptable salted forms of Compound (I) include those described in EP 0306228 and WO94/05659. A preferred pharmaceutically acceptable salt is a maleate.

Suitable pharmaceutically acceptable solvated forms of Compound (I) include those described in EP 0306228 and WO94/05659, in particular hydrates.

Compound (I) or, a pharmaceutically acceptable salt thereof, or a pharmaceutically acceptable solvate thereof, may be prepared using known methods, for example those disclosed in EP 0306228 and WO94/05659. The disclosures of EP 0306228 and WO94/05659 are incorporated herein by reference.

Compound (I) may exist in one of several tautomeric forms, all of which are encompassed by the term Compound (I) as individual tautomeric forms or as mixtures thereof. Compound (I) contains a chiral carbon atom, and hence can exist in up to two stereoisomeric forms, the term Compound (I) encompasses all of these isomeric forms whether as individual isomers or as mixtures of isomers, including racemates.

The insulin sensitisers mentioned herein are prepared in accordance with known methods, for example those disclosed in the above mentioned publications or in standard reference texts, such as the British and US Pharmacopoeias, Remington's Pharmaceutical Sciences (Mack Publishing Co.), Martindale The Extra Pharmacopoeia (London, The Pharmaceutical Press).

When used herein the term 'conditions associated with diabetes' includes those conditions associated with the pre-diabetic state, conditions associated with diabetes mellitus itself and complications associated with diabetes mellitus.

When used herein the term 'conditions associated with the pre-diabetic state' includes conditions such as insulin resistance, including hereditary insulin resistance, impaired glucose tolerance and hyperinsulinaemia.

'Conditions associated with diabetes mellitus itself include hyperglycaemia, insulin resistance, including acquired insulin resistance and obesity. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

'Complications associated with diabetes mellitus' includes renal disease, especially renal disease associated with Type II diabetes, neuropathy and retinopathy.

Renal diseases associated with Type II diabetes include nephropathy, glomerulonephritis, glomerular sclerosis, nephrotic syndrome, hypertensive nephrosclerosis and end stage renal disease.

As used herein the term 'pharmaceutically acceptable' embraces both human and veterinary use: for example the term 'pharmaceutically acceptable' embraces a veterinarily acceptable compound.

When used herein the term 'SC50 concentration' refers to the plasma concentration for a given compound required to produce a half-maximal effect on fasting plasma glucose for that compound.

For the avoidance of doubt, when reference is made herein to scalar amounts, including mg amounts, of Compound (I) in a pharmaceutically acceptable form, the scalar amount referred to is made in respect of Compound (I) *per se:* For example 2 mg of Compound (I) in the form of the maleate salt is that amount of maleate salt which contains 2 mg of Compound (I).

Diabetes mellitus is preferably Type II diabetes.

Glycaemic control may be characterised using conventional methods, for example by measurement of a typically used index of glycaemic control such as fasting plasma glucose or glycosylated haemoglobin (Hb A1c). Such indices are determined using standard methodology, for example those described in: Tuescher A, Richterich, P., Schweiz. med. Wschr. 101 (1971), 345 and 390 and Frank P., 'Monitoring the Diabetic Patent with Glycosolated Hemoglobin Measurements', Clinical Products 1988.

Preferably, the treatment of the invention will effect an improvement in the levels of advanced glycosylation end products (AGEs), leptin and serum lipids including total cholesterol, HDL-cholesterol, LDL-cholesterol including improvements in the ratios thereof, in particular an improvement in serum lipids including total cholesterol, HDL-cholesterol, LDL-cholesterol including improvements in the ratios thereof.

As indicated above, the active medicaments of the method of the invention are preferably administered in pharmaceutical composition form.

Usually the compositions are adapted for oral administration. However, they may be adapted for other modes of administration, for example parenteral administration, sublingual or transdermal administration.

The compositions may be in the form of tablets, capsules, powders, granules, lozenges, suppositories, reconstitutable powders or liquid preparations, such as oral or sterile parenteral solutions or suspensions.

In order to obtain consistency of administration it is preferred that a composition of the invention is in the form of a unit dose.

Unit dosage presentation forms for oral administration may be in tablet or capsule form and may as necessary contain conventional excipients such as binding agents, fillers, lubricants, glidants, disintegrants and wetting agents.

Examples of binding agents include acacia, alginic acid, carboxymethylcellulose calcium, carboxymethylcellulose sodium, dextrates, dextrin, dextrose, ethylcellulose, gelatin, liquid glucose, guar gum, hydroxyethyl cellulose, hydroxypropyl cellulose, hydroxypropyl methylcellulose, magnesium aluminium silicate, maltodextrin, methyl cellulose, polymethacrylates, polyvinylpyrrolidone, pregelatinised starch, sodium alginate, sorbitol, starch, syrup, tragacanth.

Examples of fillers include calcium carbonate, calcium phosphate, calcium sulphate, carboxymethylcellulose calcium, carboxymethylcellulose sodium, compressible sugar, confectioner's sugar, dextrates, dextrin, dextrose, dibasic calcium phosphate dihydrate, dibasic calcium phosphate, fructose, glyceryl palmitostearate, glycine, hydrogenated vegetable oil-type 1, kaolin, lactose, maize starch, magnesium carbonate, magnesium oxide, maltodextrin, mannitol, microcrystalline cellulose, polymethacrylates, potassium chloride, powdered cellulose, pregelatinised starch, sodium chloride, sorbitol, starch, sucrose, sugar spheres, talc, tribasic calcium phosphate, xylitol.

Examples of lubricants include calcium stearate, glyceryl monostearate, glyceryl palmitostearate, magnesium stearate, microcrystalline cellulose, sodium benzoate, sodium chloride, sodium lauryl sulphate, stearic acid, sodium stearyl fumarate, talc, zinc stearate.

Examples of glidants include colloidal silicon dioxide, powdered cellulose, magnesium trisilicate, silicon dioxide, talc.

Examples of disintegrants include alginic acid, carboxymethylcellulose calcium, carboxymethylcellulose sodium, colloidal silicon dioxide, croscarmellose sodium, crospovidone, guar gum, magnesium aluminium silicate, microcrystalline cellulose, methyl cellulose, polyvinylpyrrolidone, polacrilin potassium, pregelatinised starch, sodium alginate, sodium lauryl sulphate, sodium starch glycollate.

An example of a pharmaceutically acceptable wetting agent is sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agent can be dissolved in the vehicle. To enhance the stability, the composition can be frozen after filling into the vial and the water removed under vacuum. Parenteral suspensions are prepared in substantially the same manner, except that the Compound (I) suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

As indicated the compositions are preferably in a unit dosage form in an amount appropriate for the relevant daily dosage.

The present treatments and compositions may also contain other medicaments in addition to insulin sensitisers including other anti diabetic agents, such as insulin secretagogues, biguanide antihyperglycaemic agents and alpha glucosidase inhibitor antihyperglycaemic agents.

In the treatment the medicaments may be administered from 1 to 6 times a day, suitably 1 or 2 times per day, preferably once per day.

Compositions may contain from 0.1 % to 99% by weight, preferably from 10-60% by weight, of the active material, depending upon the method of administration.

The composition may, if desired, be in the form of a pack accompanied by written or printed instructions for use.

The method by which the Threshold Plasma Concentration, such as the SC50 concentration, for a given compound can be determined is:

1) first to obtain plasma concentrations versus time data for the compound, preferably using data from humans, by using standard pharmacokinetic compartmental modelling methods (for example for Compound (I), concentrations were fit to a one compartment model.);
2) the model predicted concentrations for the compound are then fed back into the model and used to determine the change in fasting plasma glucose levels after various doses;
3) the relationship between predicted plasma concentrations of compound and fasting plasma glucose can suitably be determined using an indirect pharmacological response model (model IV), for example that described in (Dayneka NL, Garg V and Jusko WJ, Comparison of Four Basic Models of Indirect Pharmacodynamic Responses. J of Pharmacokinetics and Biopharmaceutics. Vol 21, No 4. 1993). This model yields estimates of glucose input rate (Kin) and output rate (Kout), maximal stimulation of glucose output (Smax). Hill coefficient (gamma) and the Threshold Plasma Concentration, such as the SC50 concentration (i.e the concentration associated with a half maximal response) to be determined for that compound. This method forms a further part of the present invention.

For Compound (I), it was necessary to account for a time delay between the time of actual initiation of dosing (week 0) and the observed change in fasting plasma glucose. This delay factor was estimated through the modelling for each dose level. The mean delay across dose levels for Compound (I) was found to be 292 hours. The delay factor was incorporated into the model for deriving fasting plasma glucose by assuming that the first dose of drug occurred only after the time dictated by the delay factor. It is considered that for this model a delay factor would be required for other thiazolidinedione insulin sensitisers and that this factor will be substantially similar to that found for Compound (I). Delay factors for other compounds may also be required to be determined using similar methodology to that disclosed herein.

The invention also comprises the above mentioned method, optionally including the step of introducing the delay factor into the model.

In a further aspect, the invention provides a process for preparing a pharmaceutical composition comprising an insulin sensitiser and a pharmaceutically acceptable carrier therefor, the composition being adapted to provide a plasma concentration of the insulin sensitiser of at least a Threshold Plasma Concentration of the insulin sensitiser, which process comprises formulating the insulin sensitiser and the pharmaceutically acceptable carrier so as to provide a plasma concentration of the insulin sensitiser of at least a Threshold Plasma Concentration of the insulin sensitiser.

Suitably, the composition is a modified release composition.

Suitably, the carrier is adapted so as to provide a plasma concentration of the insulin sensitiser of at least a Threshold Plasma Concentration.

The compositions for the treatment are prepared and formulated according to conventional methods, such as those disclosed in standard reference texts, for example the British and US Pharmacopoeias, Remington's Pharmaceutical Sciences (Mack Publishing Co.), Martindale The Extra Pharmacopoeia (London, The Pharmaceutical Press) (for example see the 3 1 st Edition page 341 and pages cited therein) and Harry's Cosmeticology (Leonard Hill Books) or the above mentioned publications.

The modified release compositions may be formulated according to appropriate methods disclosed in for example Sustained and Controlled Release Drug Delivery Systems, Editor Joe R Robinson, Volume 7, published by Marcel Dekker under the title Drugs and the Pharmaceutical Sciences, Controlled Drug Delivery, 2nd Edition' edited by Joe Robinson and Vince Lee, Marcel Dekker, 1987 and 'Drug Delivery to the Gastrointestinal Tract' Editors: J G Hardy, S S. Davis and C G Wilson also with reference to texts such as the British and US Pharmacopoeias, Remington's Pharmaceutical Sciences (Mack Publishing Co.), Martindale The Extra Pharmacopoeia (London, The Pharmaceutical Press) (for example see the 3 1 st Edition page 341 and pages cited therein) and Harry's Cosmeticology (Leonard Hill Books).

No adverse toxicological effects are expected for the compositions or methods of the invention in the above mentioned dosage ranges.

**Example: Pharmacokinetic/Pharmacodynamic Modeling of Compound (I) in Type 2 Diabetes Patients**

A PK/PD model was developed to characterise the effect of Compound (I) on fasting plasma glucose (FPG) concentrations in diabetic patients. The model was developed using mean fasting plasma glucose data from a Phase III clinical trial which consisted of comparison of placebo and four doses/regimens of Compound (I) in a parallel-group design of 26 weeks duration. Dose regimens evaluated were: 4 mg once daily and 2 mg twice daily, and 8 mg once daily and 4 mg twice daily.

Pharmacokinetics of Compound (I)

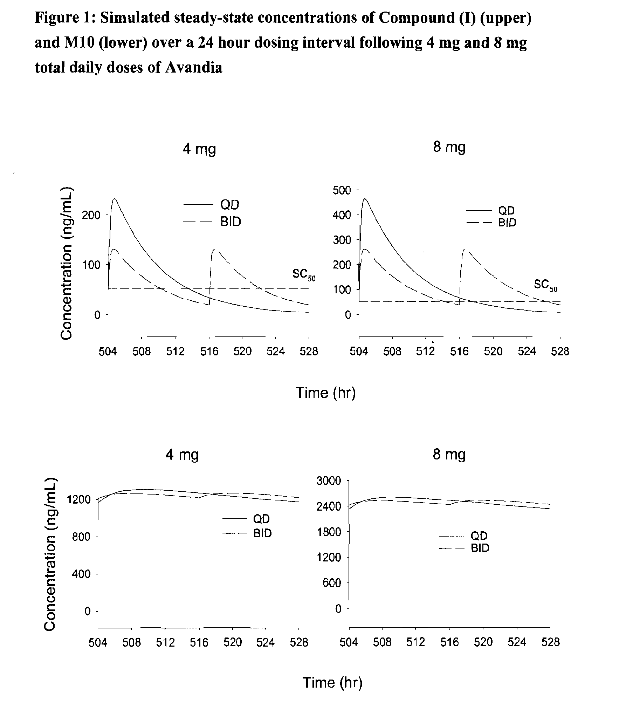

The pharmacokinetics of Compound (I) were described using a one-compartment model with first order oral absorption. Individual bayesian estimates of Compound (I) oral clearance and steady-state volume of distribution were predicted for each patient in the same Phase III clinical trial utilizing the population parameter estimates (priors) from the Phase I population pharmacokinetic analysis. Mean concentration-time profiles (Cp) for each regimen for use in the pharmacodynamic modeling were predicted using the mean post hoc oral clearance (2.68 L/h) and Vss/F (15.4 L) values across these patients (Figure 1).

Pharmacodynamics of Compound (I)

A modified indirect response model IV (Dayneka et al. 1993) was developed utilizing the pharmacokinetics of Compound (I) as the driving force for the change in fasting plasma glucose after various doses of Compound (I). Modeling fittings were done using ADAPT II, Release 4 (D'Argenio and Schumitzky, 1979).

In the absence of Compound (I), plasma glucose levels are governed by formation (kin) and utilization (kout). The action of Compound (I) was described as stimulation (S(t)) of the utilization of plasma glucose (kout), described in text as FPG reduction (Eq. 1). $S_{max}$ represents the maximal stimulation, $SC_{50}$ is the Compound (I) concentration associated with half-maximal stimulation and γ represents a sigmoidicity parameter in the Hill type function (Eq. 2).

$$\frac{dFPG}{dt} = k_{in} - k_{out} \bullet S(t) \bullet FPG \qquad\qquad Eq\ 1.$$

where

$$S(t) = 1 + \frac{S_{\max} \bullet C_P^{\gamma}}{SC_{50}^{\gamma} + C_P^{\gamma}} \qquad\qquad \text{Eq 2.}$$

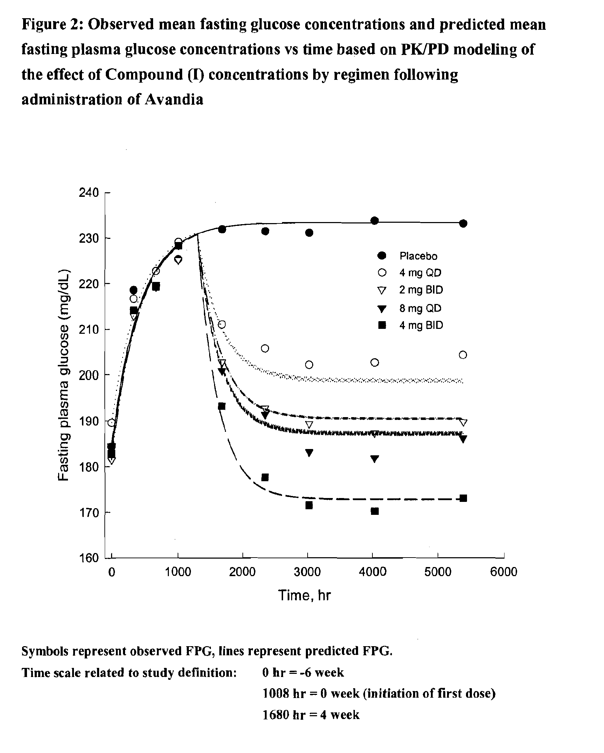

The mean fasting plasma glucose profiles from 6 weeks prior to dosing (time 0) through 26 weeks of treatment for the 5 treatment groups are shown in Figure 2. The PK/PD model accommodates the full nature of the fasting plasma glucose response over the duration of the study period as evidenced by the close agreement between observed and predicted fasting plasma glucose concentrations (Figure 2). An estimated lag-time (292 hours) between the first dose and onset of response was incorporated into the modeling. The lag-time allows the model to describe the slow onset of action of Compound (I) observed over the first 4 weeks of dosing.

The fitted plasma glucose concentrations at steady-state reflect the difference in response to varying total daily dose as well as different dosing frequencies (i.e. once vs twice daily) (Figure 2). The estimated pharmacodynamic parameter values are shown in the table below:

| Parameter | Estimate | CV % |
|---|---|---|
| kin, mg/dL of FPG per h | 0.54 | 4.5 |
| kout, $h^{-1}$ | 0.0023 | 4.5 |
| Smax | 0.44 | 7.1 |
| $SC_{50}$ | 51.4 | 10.7 |
| $\gamma$ | 3.1 | 36.6 |

Based on these data, the $S_{\max}$ of the model suggests a maximum FPG reduction of 160 mg/dL.

Although clinically meaningful reductions in glycaemia are evident with once daily dosing of Compound (I), the observation from study 024 that twice daily dosing tended to be more efficacious than once daily can be explained by differences in the concentration-time profiles of Compound (I) across these dosage regimens. Following twice daily dosing of 4 mg, Compound (I) concentrations remain above the $SC_{50}$ for approximately 21 hours compared with only 14 hours following once daily dosing with 8 mg (Figure 1).

Conclusion

The differential effects on FPG reduction following once vs twice daily dosing are well described with a PK/PD model.

References

D'Argenio, DZ and Schumitzky, A (1979). A Program Package for Simulation and Parameter Estimation in Pharmacokinetics. Computer Programs in Biomedicine. 9:115-1134.

Dayneka NL, Garg V and Jusko WJ (1993). Comparison of Four Basic Models of Indirect Pharmacodynamic Responses. Journal of Pharmacokinetics and Biopharmaceutics. Vol 21 (No. 4): 457-478.

**Claims**

1. A method for the treatment of Type 2 diabetes mellitus and conditions associated with diabetes mellitus, which method comprises the administration to a human or non-human mammal in need thereof, of an effective non-toxic amount of an insulin sensitiser so as to provide a plasma concentration of the insulin sensitiser of at least a threshold level (the 'Threshold Plasma Concentration') from within the range of effective plasma levels of the insulin sensitiser.

2. A method according to claim 1, wherein the Threshold Plasma Concentration is within the range of from about 40 to about 200ng/nL.

3. A method according to claim 1 or claim 2, wherein the Threshold Plasma Concentration is within the range of from about 50 to about 120ng/mL or about 60 to about 120ng/mL or about 90 to about 110ng/mL or about 95 to about 105ng/mL.

4. A method according to any one of claims 1 to 3, wherein a minimum value of the Threshold Plasma Concentration (or the Minimum Threshold Plasma Concentration) of the insulin sensitiser is its SC50 concentration.

5. A method according to any one of claim 1 to 4, wherein a Preferred Threshold Plasma Concentration for the insulin sensitiser is twice the SC50 concentration.

6. A method according to any one of claim 1 to 5, wherein the plasma concentration of the insulin sensitiser remains substantially within the range from the Minimum Threshold Plasma Concentration to a level at or above the Preferred Threshold Plasma Concentration.

7. A method according to any one of claim 1 to 6, wherein the insulin sensitiser is Compound (I).

8. A method according to any one of claims 4 to 6, wherein the insulin sensitiser is Compound (I) and the SC50 is within the range of 40 to 65 ng/mL.

9. A method according to claim 8, wherein the SC50 of Compound (I) is 51.4ng/mL.

10. A method according to any one of claims 6 to 9 wherein the insulin sensitiser is Compound (I) and the Preferred Threshold Plasma Concentration is in the range of about 80 to about 130 ng/mL or about 82.2 to about 123.4.

11. A method according to claim 10, wherein the Preferred Threshold Plasma Concentration for Compound (I) is 100 ng/mL or 102.8 ng/mL.

12. A method according to claim 1, wherein the insulin sensitiser is Compound (I) and its plasma concentration remains substantially within the range of from 40 ng/mL to at or above 130 ng/mL or 41.1 ng/mL to at or above 123.4 ng/mL, for example 50ng/mL to at or above 100ng/mL or 51.4ng/mL to at or above 102.8ng/mL.

13. A method according to claim 10, wherein the insulin sensitiser is Compound (I) and its plasma concentration remains substantially at or above its Preferred Threshold Plasma Concentration.

14. A method according to claim 13, wherein the insulin sensitiser is Compound (I) and its plasma concentration remains at or above 100ng/mL or substantially at or above 102.8ng/mL.

15. A method according to any one of claims 1 to 6, wherein the insulin sensitiser is 5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone), 5-[4-[(1-methylcyclohexyl)methoxy]benzyl] thiazolidine-2,4-dione (or ciglitazone), 5-[4-[2-(5-ethylpyridin-2-yl)ethoxy]benzyl] thiazolidine-2,4-dione (or pioglitazone) or 5-[(2-benzyl-2,3-dihydrobenzopyran)-5-ylmethyl)thiazolidine-2,4-dione (or englitazone).

16. A pharmaceutical composition comprising an insulin sensitiser and a pharmaceutically acceptable carrier therefor, which composition is adapted to provide a plasma concentration of the insulin sensitiser of at least a Threshold Plasma Concentration of the insulin sensitiser.

17. A pharmaceutical composition according to claim 17 wherein the composition is adapted to provide a plasma concentration of the insulin sensitiser of at least a Threshold Plasma Concentration over a sustained period of time.

18. A modified release pharmaceutical composition comprising an insulin sensitiser and a pharmaceutically acceptable carrier therefor, which composition is adapted to provide a plasma concentration of the insulin sensitiser of at least a Threshold Plasma Concentration of the insulin sensitiser.

19. A modified release composition according to claim 1 being a delayed, pulsed or sustained release composition.

20. A composition according to any one of claim 16 to 19, adapted to provide a method of treatment according to any one of claims 1 to 15.

21. A method by which the Threshold Plasma Concentration for a given anti diabetic compound can be determined by the steps:

1) first to obtain plasma concentrations versus time data for the compound by using standard pharmacokinetic compartmental modelling methods;

2) the model predicted concentrations for the compound are then fed back into the model and used to determine the change in fasting plasma glucose levels after various doses;

3) the relationship between predicted plasma concentrations of compound and fasting plasma glucose can then be determined using an indirect pharmacological response model.

**Figure 1: Simulated steady-state concentrations of Compound (I) (upper) and M10 (lower) over a 24 hour dosing interval following 4 mg and 8 mg total daily doses of Avandia**

**Figure 2: Observed mean fasting glucose concentrations and predicted mean fasting plasma glucose concentrations vs time based on PK/PD modeling of the effect of Compound (I) concentrations by regimen following administration of Avandia**

Symbols represent observed FPG, lines represent predicted FPG.

Time scale related to study definition: 0 hr = -6 week

1008 hr = 0 week (initiation of first dose)

1680 hr = 4 week

**European Patent Office**

## PARTIAL EUROPEAN SEARCH REPORT

**Application Number**

which under Rule 45 of the European Patent Convention EP 06 12 4048
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | EP 0 155 845 A (TAKEDA CHEMICAL INDUSTRIES LTD) 25 September 1985 (1985-09-25) <br> * page 8, lines 10-15 * <br> ----- | 1-21 | INV. <br> A61K31/44 <br> A61K31/425 |
| D,X | EP 0 177 353 A (TAKEDA CHEMICAL INDUSTRIES LTD) 9 April 1986 (1986-04-09) <br> * page 52, line 8 - page 53, line 8 * <br> ----- | 1-21 | |
| D,X | EP 0 428 312 A (PFIZER) 22 May 1991 (1991-05-22) <br> * page 9, lines 3-14 * <br> ----- | 1-21 | |
| D,X | EP 0 032 128 A (TAKEDA CHEMICAL INDUSTRIES LTD) 15 July 1981 (1981-07-15) <br> * page 4, lines 30-35 * <br> ----- | 1-21 | |
| D,X | EP 0 008 203 A (TAKEDA CHEMICAL INDUSTRIES LTD) 20 February 1980 (1980-02-20) <br> * page 4, lines 20-26 * <br> ----- | 1-21 | |
| D,X | EP 0 489 663 A (TERUMO CORP) 10 June 1992 (1992-06-10) <br> * page 4, lines 47-53 * <br> ----- | 1-21 | **TECHNICAL FIELDS SEARCHED (IPC)** <br><br> A61K |

### INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 11 January 2007 | Cattell, James |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                     

& : member of the same patent family, corresponding document

EPO FORM 1503 03.82 (P04C07)

European Patent
Office

INCOMPLETE SEARCH
SHEET C

Application Number

EP 06 12 4048

Claim(s) searched completely:
        16-20

Claim(s) searched incompletely:
        1-15, 21

Reason for the limitation of the search (non-patentable invention(s)):

Article 52 (4) EPC - Method for treatment of the human or animal body by surgery

        -----

Further limitation of the search

Claim(s) searched incompletely:
        1-21

Reason for the limitation of the search:

Present claims 1-21 relate to a dosage concentration defined by reference to the acheived parameter of "threshold plasma concentration".

The use of this parameter in the present context is considered to lead to a lack of clarity within the meaning of Article 84 EPC. It is impossible to compare the parameters the Applicant has chosen to employ with what is set out in the prior art. The lack of clarity is such as to render a meaningful complete search impossible.

Consequently, the search has been restricted to the concentration that give the threshold plasma concentrations, defined as being "suitable unit dosages" indicated in the cited prior art on page 3, lines 24 to 27 of the present description.

## ANNEX TO THE EUROPEAN SEARCH REPORT
## ON EUROPEAN PATENT APPLICATION NO.

EP 06 12 4048

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2007

| Patent document cited in search report | | Publication date | Patent family member(s) | | Publication date |
|---|---|---|---|---|---|
| EP 0155845 | A | 25-09-1985 | CA | 1263961 A1 | 19-12-1989 |
| | | | US | 4582839 A | 15-04-1986 |
| EP 0177353 | A | 09-04-1986 | AU | 583537 B2 | 04-05-1989 |
| | | | AU | 4817685 A | 10-04-1986 |
| | | | CA | 1263655 A1 | 05-12-1989 |
| | | | CN | 85107870 A | 16-07-1986 |
| | | | DE | 3577092 D1 | 17-05-1990 |
| | | | DK | 444685 A | 04-04-1986 |
| | | | ES | 8706150 A1 | 16-08-1987 |
| | | | ES | 8801256 A1 | 01-03-1988 |
| | | | FI | 853796 A | 04-04-1986 |
| | | | GR | 852389 A1 | 04-02-1986 |
| | | | JP | 1859452 C | 27-07-1994 |
| | | | JP | 61085372 A | 30-04-1986 |
| | | | NO | 853902 A | 04-04-1986 |
| | | | PT | 81235 A | 01-11-1985 |
| | | | US | 4725610 A | 16-02-1988 |
| EP 0428312 | A | 22-05-1991 | AT | 116306 T | 15-01-1995 |
| | | | CA | 2029703 A1 | 14-05-1991 |
| | | | DE | 69015587 D1 | 09-02-1995 |
| | | | DE | 69015587 T2 | 04-05-1995 |
| | | | DK | 428312 T3 | 20-03-1995 |
| | | | ES | 2066991 T3 | 16-03-1995 |
| | | | FI | 922141 A | 12-05-1992 |
| | | | GR | 3015041 T3 | 31-05-1995 |
| | | | IE | 904057 A1 | 22-05-1991 |
| | | | JP | 1975564 C | 27-09-1995 |
| | | | JP | 3170478 A | 24-07-1991 |
| | | | JP | 7008862 B | 01-02-1995 |
| | | | PT | 95852 A | 13-09-1991 |
| | | | WO | 9107107 A1 | 30-05-1991 |
| EP 0032128 | A | 15-07-1981 | AR | 228444 A1 | 15-03-1983 |
| | | | DK | 2281 A | 08-07-1981 |
| | | | ES | 8202546 A1 | 01-05-1982 |
| | | | JP | 1463862 C | 28-10-1988 |
| | | | JP | 56097277 A | 05-08-1981 |
| | | | JP | 63010702 B | 08-03-1988 |
| | | | US | 4376777 A | 15-03-1983 |
| EP 0008203 | A | 20-02-1980 | CA | 1131644 A1 | 14-09-1982 |
| | | | DE | 2963585 D1 | 21-10-1982 |
| | | | DK | 325079 A | 05-02-1980 |
| | | | ES | 8102107 A1 | 01-04-1981 |

EPO FORM P0459

For more details about this annex : see Official Journal of the European Patent Office, No. 12/82

**EP 1 759 698 A1**

## ANNEX TO THE EUROPEAN SEARCH REPORT
## ON EUROPEAN PATENT APPLICATION NO.

EP 06 12 4048

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2007

| Patent document cited in search report | | Publication date | Patent family member(s) | | Publication date |
|---|---|---|---|---|---|
| EP 0008203 | A | | JP | 1433701 C | 07-04-1988 |
| | | | JP | 55022636 A | 18-02-1980 |
| | | | JP | 62042903 B | 10-09-1987 |
| | | | US | 4287200 A | 01-09-1981 |
| | | | US | 4340605 A | 20-07-1982 |
| | | | US | 4438141 A | 20-03-1984 |
| | | | US | 4444779 A | 24-04-1984 |
| EP 0489663 | A | 10-06-1992 | JP | 4210683 A | 31-07-1992 |
| | | | US | 5225426 A | 06-07-1993 |

EPO FORM P0459

For more details about this annex : see Official Journal of the European Patent Office, No. 12/82

15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0306228 A
- WO 9405659 A
- EP 0008203 A
- EP 0139421 A
- EP 0032128 A
- EP 0428312 A
- EP 0489663 A
- EP 0155845 A
- EP 0257781 A
- EP 0208420 A
- EP 0177353 A
- EP 0319189 A
- EP 0332331 A
- EP 0332332 A
- EP 0528734 A

- EP 0508740 A
- EP 9218501 A
- EP 9302079 A
- EP 9322445 A
- US 5104888 A
- US 5478852 A
- WO 9321166 A
- WO 9401420 A
- US 5232945 A
- WO 9203425 A
- WO 9119702 A
- EP 0533933 A
- JP 05271204 B
- US 5264451 A

**Non-patent literature cited in the description**

- **TUESCHER A ; RICHTERICH, P.** *Schweiz. med. Wschr.,* 1971, vol. 101, 345, 390
- **FRANK P.** Monitoring the Diabetic Patent with Glycosolated Hemoglobin Measurements. *Clinical Products,* 1988
- **DAYNEKA NL ; GARG V ; JUSKO WJ.** Comparison of Four Basic Models of Indirect Pharmacodynamic Responses. *J of Pharmacokinetics and Biopharmaceutics,* 1993, vol. 21 (4
- British and US Pharmacopoeias, Remington's Pharmaceutical Sciences. Mack Publishing Co,
- Martindale The Extra Pharmacopoeia. The Pharmaceutical Press, 341
- Sustained and Controlled Release Drug Delivery Systems. Marcel Dekker, vol. 7

- Drugs and the Pharmaceutical Sciences, Controlled Drug Delivery. Marcel Dekker, 1987
- Drug Delivery to the Gastrointestinal Tract. British and US Pharmacopoeias, Remington's Pharmaceutical Sciences. Mack Publishing Co,
- Harry's Cosmeticology. Leonard Hill Books
- **D'ARGENIO, DZ ; SCHUMITZKY, A.** A Program Package for Simulation and Parameter Estimation in Pharmacokinetics. *Computer Programs in Biomedicine.,* 1979, vol. 9, 115-1134
- **DAYNEKA NL ; GARG V ; JUSKO WJ.** Comparison of Four Basic Models of Indirect Pharmacodynamic Responses. *Journal of Pharmacokinetics and Biopharmaceutics.,* 1993, vol. 21 (4), 457-478